(19) Europäisches Patentamt
European Patent Office
Office européen des brevets

(11) Veröffentlichungsnummer: **0 593 930 A1**

# (12) EUROPÄISCHE PATENTANMELDUNG

(21) Anmeldenummer: **93115359.7**

(51) Int. Cl.[5]: **C07D 215/48**

(22) Anmeldetag: **23.09.93**

(30) Priorität: **06.10.92 DE 4233601**

(43) Veröffentlichungstag der Anmeldung:
**27.04.94 Patentblatt 94/17**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Stoltefuss, Jürgen, Dipl.-Ing.**
**Parkstrasse 20**
**D-42781 Haan(DE)**

## (54) 3-Substituierte Chinolin-5-carbonsäuren.

(57) Die vorliegende Erfindung betrifft neue 3-substituierte Chinolin-5-carbonsäuren der allgemeinen Formel (I)

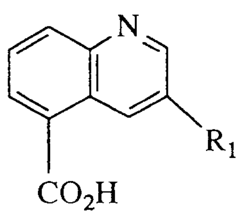

(I)

in welcher $R^1$ die in der Beschreibung angegebene Bedeutung hat, ein Verfahren zu ihrer Herstellung und ihre Verwendung bei der Herstellung von 4-Chinolyl-dihydropyridinen.

EP 0 593 930 A1

Die vorliegende Erfindung betrifft neue 3-substituierte Chinolin-5-carbonsäuren, ein Verfahren zu ihrer Herstellung und ihre Verwendung bei der Herstellung von 4-Chinolyl-dihydropyridinen.

Aus der Publikation DE 40 298 07 ist die Verbindung 3-Phenyl-5-chinolincarbonsäure bekannt, während die entsprechende 3-Methyl-substituierte Verbindung in der JP 59 184 161 aufgeführt ist.

Außerdem werden einige erfindungsgemäße Verbindungen von dem Bedeutungsumfang eines Zwischenproduktes der EP 452 712 umfaßt, ohne daß dort ein konkreter Stoffvertreter genannt wird.

Die vorliegende Erfindung betrifft neue 3-substituierte Chinolin-5-carbonsäuren der allgemeinen Formel (I)

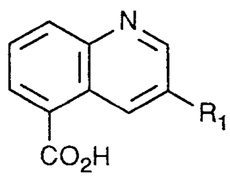

(I)

in welcher

$R^1$ für geradkettiges oder verzweigtes Alkyl mit 2 bis 8 Kohlenstoffatomen steht, oder für einen Rest der Formel $-A-R^2$ steht, worin

A eine einfache Bindung, ein Sauerstoff- oder Schwefelatom oder eine Alkylidenkette mit bis zu 3 C-Atomen bedeutet, und

$R^2$ Pyridyl oder Phenyl bedeutet, das gegebenenfalls bis zu 3-fach gleich oder verschieden durch Nitro, Halogen, Trifluormethyl oder durch geradkettiges oder verzweigtes Alkyl, Alkoxy oder Alkoxycarbonyl mit jeweils bis zu 8 Kohlenstoffatomen, Hydroxy oder Carboxy substituiert ist, wobei für den Fall, daß A für eine einfache Bindung steht, $R^2$ nicht für unsubstituiertes Phenyl steht,

und deren Salze.

Bevorzugt sind Verbindungen der allgemeinen Formel (I),

in welcher

$R^1$ für geradkettiges oder verzweigtes Alkyl mit 2 bis zu 6 Kohlenstoffatomen steht, oder für einen Rest der Formel $-A-R^2$ steht,

worin

A eine einfache Bindung, ein Sauerstoff- oder Schwefelatom oder eine Alkylidenkette mit bis zu 2 C-Atomen bedeutet

und

$R^2$ Pyridyl oder Phenyl bedeutet, das gegebenenfalls bis zu 2-fach gleich oder verschieden durch Nitro, Fluor, Chlor, Brom, Trifluormethyl oder durch geradkettiges oder verzweigtes Alkyl, Alkoxy oder Alkoxycarbonyl mit jeweils bis zu 6 Kohlenstoffatomen substituiert ist, wobei für den Fall, daß A für eine einfache Bindung steht, $R^2$ nicht für unsubstituiertes Phenyl steht,

und deren Salze.

Besonders bevorzugt sind Verbindungen der allgemeinen Formel (I),

in welcher

$R^1$ für geradkettiges oder verzweigtes Alkyl mit 2 bis 4 Kohlenstoffatomen steht, oder für einen Rest der Formel $-A-R^2$ steht, worin

A eine einfache Bindung, ein Sauerstoff- oder Schwefelatom oder die $-CH_2$-Gruppe bedeutet und

$R^2$ Pyridyl oder Phenyl bedeutet, das gegebenenfalls bis zu 2-fach gleich oder verschieden durch Nitro, Fluor, Chlor, Trifluormethyl oder durch geradkettiges oder verzweigtes Alkyl, Alkoxy oder Alkoxycarbonyl mit jeweils bis zu 4 Kohlenstoffatomen substituiert ist, wobei für den Fall, daß A für eine einfache Bindung steht, $R^2$ nicht für unsubstituiertes Phenyl steht,

und deren Salze.

Ganz besonders bevorzugt sind Verbindungen der allgemeinen Formel (I), in welcher

$R^1$ für n-Butyl, i-Propyl oder tert.Butyl steht, oder für einen Rest der Formel -A-$R^2$ steht, worin

A eine einfache Bindung, ein Sauerstoff- oder Schwefelatom oder die -$CH_2$-Gruppe bedeutet und

$R^2$ Pyridyl oder Phenyl bedeutet, das gegebenenfalls bis zu 2-fach gleich oder verschieden durch Fluor, Chlor, Nitro, Methyl, Methoxy oder Methoxycarbonyl substituiert ist, wobei für den Fall, daß A für eine einfache Bindung steht, $R^2$ nicht für unsubstituiertes Phenyl steht,

und deren Salze.

Außerdem wurde ein Verfahren zur Herstellung der erfindungsgemäßen Verbindungen der allgemeinen Formel (I) gefunden, dadurch gekennzeichnet, daß man 4-Amino-3-hydroxyphthalid der Formel (II)

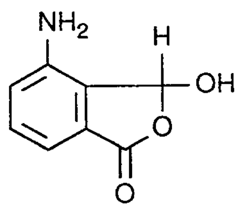

(II)

mit Aldehyden der allgemeinen Formel (III)

$R^1$-$CH_2$-CHO (III)

in welchen $R^1$ die oben angegebene Bedeutung hat, in inerten Lösemitteln umsetzt.

Das erfindungsgemäße Verfahren kann durch folgendes Formelschema beispielhaft erläutert werden:

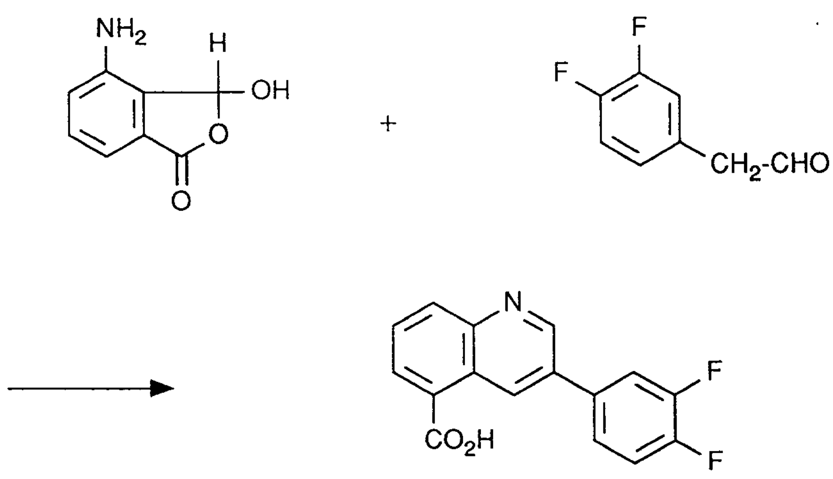

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol, sowie Ester wie beispielsweise Essigsäureethylester. Ebenso ist es möglich, Gemische der genannten Lösemittel zu verwenden. Bevorzugt sind Methanol, Isopropanol, Ethanol und n-Propanol, Acetonitril, Tetrahydrofuran oder Diethylenglykoldimethylether.

Die Reaktionstemperaturen können in einem größeren Bereich variiert werden. Im allgemeinen arbeitet man zwischen +20 °C und +150 °C, vorzugsweise zwischen 60 °C und 120 °C, insbesondere bei der Siedetemperatur des betreffenden Lösemittels.

Die Umsetzung kann bei Normaldruck, aber auch bei erhöhtem Druck (z.B. 1 bis 50 bar) durchgeführt werden. Im allgemeinen arbeitet man bei Normaldruck.

Die Verbindung der Formel (II) ist bekannt [vgl. DE 40 298 07].

Die Aldehyde der allgemeinen Formel (III) sind teilweise bekannt oder können nach bekannten Methoden hergestellt werden.

Das vorstehende Herstellungsverfahren ist lediglich zur Verdeutlichung angegeben. Die Herstellung der erfindungsgemäße Verbindung der Formel (I) ist nicht auf dieses Verfahren beschränkt, sondern jede Modifikation dieses Verfahren, beispielsweise die Anwendung literaturbekannnter Nitro-Amino-Reduktionsmethoden, sind in gleicher Weise für die Herstellung der erfindungsgemäßen Verbindung anwendbar.

Die erfindungsgemäßen Verbindungen sind für die 1,4-Dihydropyridin-Chemie von großer Bedeutung, da sie wertvolle Zwischenprodukte zur Synthese von 4-Chinolyl-dihydropyridinen sind [vgl. hierzu z.B. EP 452 712].

Ausgangsverbindungen

Beispiel Z1

4-Amino-3-hydroxyphthalid

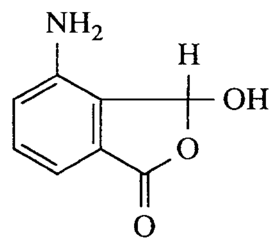

10 g 3-Hydroxy-4-nitro-phthalid werden in 100 ml Tetrahydrofuran gelöst und nach Zugabe von 1 g Palladium auf Bariumsulfat (5%) bei Atmosphärendruck und 20 - 50 °C hydriert. Es wird vom Katalysator abfiltriert und eingeengt. Der Eindampfrückstand wird mit Ether verrührt und abgesaugt. Man erhält 5,8 g (68,5% der Theorie) einer farblosen Substanz vom Schmp. 280-285 °C (Zers.).

Herstellungsbeispiele

Beispiel 1

3-Phenoxy-chinolin-5-carbonsäure

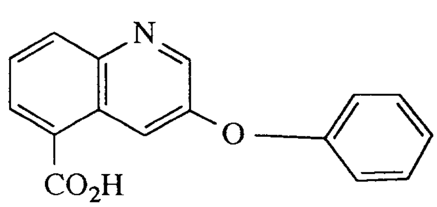

50 g (0,256 mol) 3-Hydroxy-4-nitro-phthalid werden in 250 ml Ethanol mit 5 g Palladium/Bariumsulfat (5%) bei 40 °C bis 50 °C und 3,5 bar hydriert. Es wird abgesaugt, das Filtrat wird mit 45 g (0,33 mol) Phenoxyacetaldehyd versetzt und 20 Stunden gekocht. Das ausgefallene Produkt wird nach dem Abkühlen abgesaugt und mit Ethanol gewaschen. Man erhält 25,5 g (36,85% der Theorie) vom Schmp. >250 °C.

In Analogie zur Vorschrift des Beispiels 1 werden die in Tabelle 1 aufgeführten Beispiele hergestellt:

<u>Tabelle 1:</u>

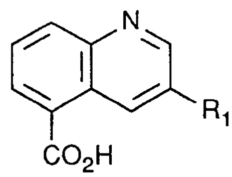

| Bsp.-Nr. | $R^1$ | F (°C) |
|---|---|---|
| 2 | 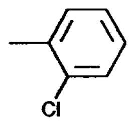 | >280 |
| 3 | 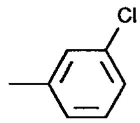 | >280 |
| 4 | 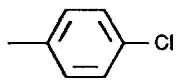 | >280 |
| 5 | 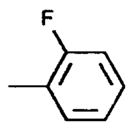 | >280 |
| 6 | 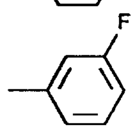 | >280 |
| 7 | 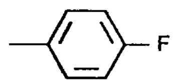 | 336 (zers.) |
| 8 | 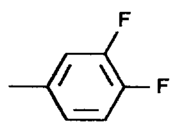 | >280 |
| 9 | 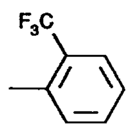 | >280 |
| 10 | 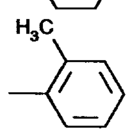 | >280 |
| 11 | 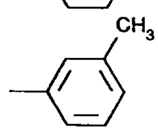 | >250 |

Fortsetzung Tabelle 1:

| Bsp.-Nr. | $R^1$ | F (°C) |
|---|---|---|
| 12 | 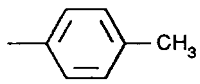 | >280 |
| 13 | 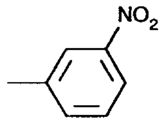 | >280 |
| 14 | 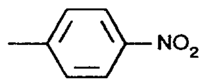 | >250 |
| 15 | 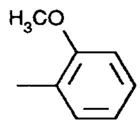 | >250 |
| 16 | 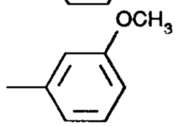 | 186-188 (Zers.) |
| 17 | 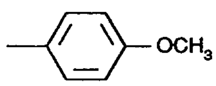 | >250 |
| 18 | 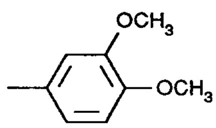 | 287-290 (Zers.) |
| 19 | 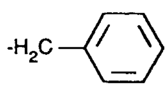 | 278-280 |
| 20 | 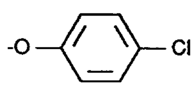 | 290-291 |
| 21 | 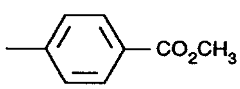 | >280 |
| 22 | 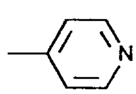 | >250 |
| 23 | 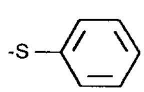 | 287-288 |

Fortsetzung Tabelle 1:

| Bsp.-Nr. | $R^1$ | F (°C) |
|---|---|---|
| 24 | 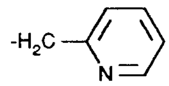 | 190 |
| 25 | 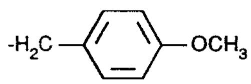 | 285-286 |
| 26 | $-C(CH_3)_3$ | 150 |
| 27 | $-n-C_4H_9$ | 230 (Zers.) |
| 28 | 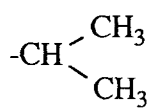 | 284-86 |

**Patentansprüche**

1. 3-substituierte-Chinolin-5-carbonsäuren der allgemeinen Formel (I)

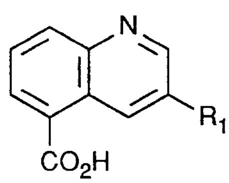

(I)

in welcher

$R^1$ für geradkettiges oder verzweigtes Alkyl mit 2 bis 8 Kohlenstoffatomen steht, oder für einen Rest der Formel $-A-R^2$ steht,

worin

A eine einfache Bindung, ein Sauerstoff- oder Schwefelatom oder eine Alkylidenkette mit bis zu 3 C-Atomen bedeutet,

und

$R^2$ Pyridyl oder Phenyl bedeutet, das gegebenenfalls bis zu 3-fach gleich oder verschieden durch Nitro, Halogen, Trifluormethyl oder durch geradkettiges oder verzweigtes Alkyl, Alkoxy oder Alkoxycarbonyl mit jeweils bis zu 8 Kohlenstoffatomen, Hydroxy oder Carboxy substituiert ist,

wobei für den Fall, daß A für eine einfache Bindung steht, $R^2$ nicht für unsubstituiertes Phenyl steht,

und deren Salze.

2. Verbindungen der allgemeinen Formel (I) gemäß Anspruch 1,

in welcher

$R^1$ für geradkettiges oder verzweigtes Alkyl mit 2 bis zu 6 Kohlenstoffatomen steht, oder

für einen Rest der Formel -A-$R^2$ steht,

worin

A eine einfache Bindung, ein Sauerstoff- oder Schwefelatom oder eine Alkylidenkette mit bis zu 2 C-Atomen bedeutet

und

$R^2$ Pyridyl oder Phenyl bedeutet, das gegebenenfalls bis zu 2-fach gleich oder verschieden durch Nitro, Fluor, Chlor, Brom, Trifluormethyl oder durch geradkettiges oder verzweigtes Alkyl, Alkoxy oder Alkoxycarbonyl mit jeweils bis zu 6 Kohlenstoffatomen substituiert ist, wobei für den Fall, daß A für eine einfache Bindung steht, $R^2$ nicht für unsubstituiertes Phenyl steht,

und deren Salze.

**3.** Verbindungen der allgemeinen Formel (I) gemäß Anspruch 1,
in welcher

$R^1$ für geradkettiges oder verzweigtes Alkyl mit 2 bis 4 Kohlenstoffatomen steht, oder für einen Rest der Formel -A-$R^2$ steht,

worin

A eine einfache Bindung, ein Sauerstoff- oder Schwefelatom oder die $-CH_2$-Gruppe bedeutet

und

$R^2$ Pyridyl oder Phenyl bedeutet, das gegebenenfalls bis zu 2-fach gleich oder verschieden durch Nitro, Fluor, Chlor, Trifluormethyl oder durch geradkettiges oder verzweigtes Alkyl, Alkoxy oder Alkoxycarbonyl mit jeweils bis zu 4 Kohlenstoffatomen substituiert ist, wobei für den Fall, daß A für eine einfache Bindung steht, $R^2$ nicht für unsubstituiertes Phenyl steht,

und deren Salze.

**4.** Verbindungen der allgemeine Formel (I) gemäß Anspruch 1,
in welcher

$R^1$ für n-Butyl, i-Propyl oder tert.Butyl steht, oder für einen Rest der Formel -A-$R^2$ steht,

worin

A eine einfache Bindung, ein Sauerstoff- oder Schwefelatom oder die $-CH_2$-Gruppe bedeutet

und

$R^2$ Pyridyl oder Phenyl bedeutet, das gegebenenfalls bis zu 2-fach gleich oder verschieden durch Fluor, Chlor, Nitro, Methyl, Methoxy oder Methoxycarbonyl substituiert ist, wobei für den Fall, daß A für eine einfache Bindung steht, $R^2$ nicht für unsubstituiertes Phenyl steht,

und deren Salze.

**5.** Verfahren zur Herstellung von Verbindungen der allgemeinen Formel (I)

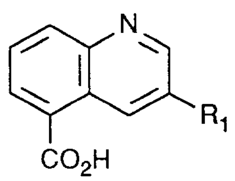

(I)

in welcher

$R^1$ für geradkettiges oder verzweigtes Alkyl mit 2 bis 8 Kohlenstoffatomen steht, oder für einen Rest der Formel -A-$R^2$ steht,

worin

A für eine einfache Bindung, ein Sauerstoff- oder Schwefelatom oder eine Alkylidenkette mit bis zu 3 C-Atomen bedeutet,

und

$R^2$ Pyridyl oder Phenyl bedeutet, das gegebenenfalls bis zu 3-fach gleich oder verschieden durch Nitro, Halogen, Trifluormethyl oder durch geradkettiges oder verzweigtes Alkyl, Alkoxy oder Alkoxycarbonyl mit jeweils bis zu 8 Kohlenstoffatomen, Hydroxy oder Carboxy substituiert ist,

wobei für den Fall, daß A für eine einfache Bindung steht, $R^2$ nicht für unsubstituiertes Phenyl steht,

und deren Salze,

dadurch gekennzeichnet, daß man 4-Amino-3-hydroxyphthalid der Formel (II)

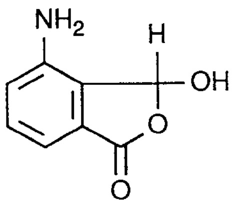

(II)

mit Aldehyden der allgemeinen Formel (III)

$R^1$-$CH_2$-CHO (III)

in welchen

$R^1$ die oben angegebene Bedeutung hat,

in inerten Lösemitteln bei Temperaturen zwischen 20 und 150 °C umsetzt.

**6.** Verwendung von Verbindungen der allgemeinen Formel (I) gemäß Anspruch 1 bei der Synthese von 4-Chinolyl-dihydropyridinen.

Europäisches Patentamt

# EUROPÄISCHER RECHERCHENBERICHT

Nummer der Anmeldung
EP 93 11 5359

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | EP-A-0 452 712 (BAYER AG) 23. Oktober 1991 * Seite 11, Zeile 10 - Seite 13, Zeile 8; Anspruch 6; Beispiel II * | 1-6 | C07D215/48 |
| D,A | EP-A-0 476 474 (BAYER AG) 25. März 1992 * Beispiel 2 * | 1-6 | |
| D,A | DATABASE WPI Week 8448, Derwent Publications Ltd., London, GB; AN 84-297394 & JP-A-59 184 161 (MITSUBISHI CHEM. IND. K.K. & TOKYO KASEI KOGYO K.K.) 19. Oktober 1984 * Zusammenfassung * | 1-6 | |

RECHERCHIERTE SACHGEBIETE (Int. Cl.5 )

C07D

Der vorliegende Recherchenbericht wurde für alle Patentansprüche erstellt

| Recherchenort | Abschlußdatum der Recherche | Prüfer |
|---|---|---|
| MUENCHEN | 11 FEBRUAR 1994 | HERZ C.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)